# EUROPEAN PATENT APPLICATION

(11) **EP 1 747 776 A1**
(43) Date of publication of application: **31.01.2007**
(21) Application number: 05016555.4
(22) Date of filing: 29.07.2005
(51) Int. Cl.: A61K 9/16, A61K 9/28, A61K 31/4439

(54) **Pharmaceutical composition comprising granular pantoprazole**

(71) Applicant: KRKA, tovarna zdravil, d.d., Novo mesto, 8501 Novo mesto (SI)
(72) Inventor: Vrecer, Franc, 8351 Straza pri Novem mestum (SI); Ferlan, Andrej, 1275 Smartno pri Litiji (SI); Kramar, Andrejka, 8000 Novo mesto (SI); Turk, Urska, 8000 Novo mesto (SI); Cernosa, Lidia, 8311 Kostanjevica na Krki (SI); Breznik, Marjanca, 1000 Ljubljana (SI); Ritlop, Gregor, 2000 Maribor (SI)
(74) Representative: UEXKÜLL & STOLBERG

(57) **Abstract**

Pharmaceutical composition comprising pantoprazole as the active ingredient. The pantoprazole is present in the form of particles having an average particle size within the range of 60 μm to 250 μm. Pantoprazole having an average particle size within the range of 60 μm to 250 μm is prepared by a process comprising the steps of wetting pantoprazole with water having a pH of 10 to 12 in a high shear mixer, passing the wet pantoprazole through a sieve having a mesh size of 0.8 to 2 mm, and drying the mass in a fluid bed dryer.

## Description

### Field of the invention

The present invention relates to pharmaceutical compositions which comprise granular pantoprazole as active ingredient and a method for the preparation or granular pantoprozole.

### Background of the invention

Pantoprazole (5-(difluoromethoxy)-2{(3,4-di-methoxy-2-pyridinyl)-methyl]-sulphinyl}-H-benz-imidazole) is a proton pump inhibitor belonging to the group of benzimidazoles. This compound inhibits gastric acid formation and thereby it is very efficient for the treatment of gastric and duodenum ulcers, gastro-oesophageal reflux and the like. It inhibits gastric acid secretion by inhibiting H⁺-K⁺ ATPase (proton-pump) activity. Pantoprazole and physiologically acceptable salts thereof are known from EP 0 166 287 and Kohl et al., The Journal of Medicinal Chemistry; 1992, 35, No. 6, 1049-1057.

Pantoprazole is known to be sensitive to heat and unstable in an acidic environment as well as in the presence of moisture and organic solvents. Thus, pharmaceutical compositions comprising this drug, as well as processes for the manufacture thereof, must be designed to protect the drug from moisture and acidic conditions. Due to the rapid drug degradation that occurs in acidic gastric fluids, such formulations usually comprise an enteric coating.

The stability problems associated with benzimidazole compounds are well known in the art, which teaches various measures for preparing stable formulations containing benzimidazole compounds. The most common approach is the use of an alkaline core, a separating layer and an enteric coating. By use of an alkaline substance within the core benzimidazole compounds can be protected against acid degradation.

EP 0 244 380 discloses pharmaceutical preparations containing acid labile compounds. The preparations consist of a core containing either the active ingredient together with an alkaline reacting compound or an alkaline salt of the active ingredient, one or more water-soluble sub-coating layers which may also contain an alkaline component and an enteric coating layer.

According to WO 92/22284 certain ingredients such as lactose, microcrystalline cellulose and hydroxypropyl cellulose being present in the compositions of EP 0 244 380 accelerate the decomposition of the pantoprazole. It is said that more stable formulations could be obtained by using polyvinylpyrrolidone and/or hydroxypropyl-methyl cellulose as binder and mannitol as an optional inert filler in the tablet core.

WO 2005/051348, on the other hand, discloses that hygroscopic polymeric binders such as polyvinylpyrrolidone impair the release of the active ingredient after storage due to absorbed humidity. It was found that by using appropriately chosen excipients compatible with the active ingredient the use of a polymeric binder can be avoided. This document teaches the use of tablet cores containing pantoprazole as active ingredient, spray-dried or granulated mannitol having an average particle size between 100 µm and 500 µm, an alkalizing substance, a disintegrant and a lubricant. The active ingredient can be granulated with an aqueous solution of mannitol in order to improve its flowability. It is known that drying of granulates based on sugar alcohols is difficult and time consuming and requires a high energy input. Furthermore, prolonged drying times are disadvantageous in view of the heat sensitivity of pantoprazole.

### Description of the Invention

It is the object of the present invention to provide tablets comprising pantoprazole as the active ingredient which are stable and which can be prepared by a direct compression process. It is a further object of the present invention to provide a process for granulating pantoprazole which does not require excessive heating.

It was found by the present inventors that this object can be achieved by using pantoprazole in the form of particles having an average particle size of 60 to 250 µm, preferably 100 to 250 µm, more preferably 125 to 230 µm and most preferably 140 to 200 µm. If pantoprazole having average particle size in the range 60 to 250 µm is mixed with other ingredients such as alkalizing substances, fillers and disintegrants, the mixture can easily be formed into tablet cores by direct compression thereof without the addition of water. Pantoprazole which is commercially may have an average particle size of as little as 25 µm.

This finding is surprising since pantoprazole exhibits inappropriate physical properties for direct compression. Furthermore, the tablet cores usually contain 20 to 50 % by weight, preferably 25 to 30 % by weight of pantoprazole. Due to the relatively high amount of the active ingredient the addition of other excipients which are better suited for direct compression is limited. If the size of the tablet cores exceeds a certain weight the disintegration of the tablets within the small intestine would be delayed and the pharmacological effect of the active ingredient would be diminished.

The term "average particle size" as used herein refers to the volume mean diameter of particles. The volume mean diameter can be determined by laser light scattering using e.g. a Malvern-Mastersizer Apparatus MS 2000. Particle sizes are determined by measuring the angular distribution of laser light scattered by a homogeneous suspension of particles. The size distribution is determined from the light scattering data using the theory of light scattering developed by Gustav Mie.

In addition to pantoprazole, the pantoprazole particles may contain further additives but it is preferred that they essentially and more preferably completely consists of pantoprazole or pharmaceutically acceptable a salt thereof.

The compositions according to the invention contain as active ingredient pantoprazole, preferably in the form of a pharmaceutically acceptable salt thereof such as the sodium salt of pantoprazole, more preferably the sesquihydrate of the sodium salt of pantoprazole.

The compositions preferably have the form of a tablet comprising
(a) a core comprising the active ingredient, an alkalizing substance, a filler and a disintegrant;
(b) an optional water-soluble separating layer or subcoating; and
(c) an enteric coating.

An additional water-soluble protecting layer may be applied to the enteric coating layer.

The tablets according to the invention contain as the alkalizing agent preferably an alkali or earth alkali hydroxide, an alkali or earth alkali carbonate, an alkali or earth alkali hydrogen carbonate, an alkali phosphate, such as sodium phosphate, a glucosamine or a mixture of two or more of these compounds, preferably sodium hydroxide, sodium carbonate, sodium hydrogen carbonate, or a mixture of two or more of these components, more preferably sodium carbonate.

As filler the tablets preferably contain lactose or a sugar alcohol, such as mannitol, sorbitol, maltitol, erythrol, or a mixture of two or more of these components. The most preferred fillers are mannitol and sorbitol and mixtures thereof. It is preferred that the filler has an average particle size of 30 to 500 µm, more preferably an average particle size of 250 to 500 µm. Mannitol is known for its non-hygroscopicity while sorbitol which is an isomer of mannitol is hygroscopic. Mixtures of mannitol and sorbitol proved to be particularly suitable as a filler. This finding is surprising since hygroscopic additives are said to impair the release of pantoprazole after storage. Tablets prepared according to the present invention did not show a significant degrease in the dissolution of the active ingredient after storage even if sorbitol was used as the only filler. It is preferred to use mixtures comprising 1 part by weight of mannitol and 1 to 5 parts by weight of sorbitol, more preferably 2 to 4 parts by weight of sorbitol and most preferably 2.8 to 3.8 parts by weight of sorbitol.

Preferred disintegrants are crospovidone, sodium starch glycolate, cross-linked carboxylic methylcelluloses, such as croscarmelose, sodium, sodium carboxymethyl starch, sodium carboxymethyl cellulose, potassium carboxymethyl cellulose, or a mixture of two or more of these components. Crospovidone is the most preferred disintegrant. Crospovidone is cross-linked polyvinyl pyrrolidone.

Pantoprazole containing tablets should disintegrate rapidly when leaving the stomach and reaching the upper part of the intestine where the physiological pH raises to values higher than 5.5 and where the polymer of the enteric coating dissolves. It has surprisingly been found that the disintegration of coated tablets can be improved by using a disintegrant having a defined particles size. By using a combination of disintegrant with an average particle size 10 to 60 µm (Type A), preferably crospovidone, and disintegrant with an average particle size 80 to 200 µm (Type B), preferably crospovidone, the disintegration time can be significantly reduced. Mixtures comprising 1 part by weight of disintegrant Type A and 1 to 5 parts by weight of disintegrant Type B, preferably 2 to 4 parts by weight of disintegrant of Type B proved to be particularly advantageous.

The tablet cores preferably also contain a lubricant. Preferred lubricants are stearic acid, hydrogenated vegetable oils, paraffin or alkali or alkaline earth stearates, aluminum stearate, sodium stearyl fumarate or mixtures of two or more of these compounds. The most preferred lubricants are magnesium stearate and calcium stearate.

The tablet cores may also comprise a binder, such as microcrystalline cellulose, hydroxypropyl methyl cellulose, polyvinylpyrrolidone (povidone) or mixtures of two or more of these compounds.

The tablet cores preferably comprise
15 to 50 % by weight, preferably 20 to 30 % by weight, more preferably 25 to 30 % by weight of the active ingredient,
2 to 25 % by weight, preferably 5 to 20 % by weight, more preferably 5 to 15 % by weight of the alkalizing substance,
10 to 50 % by weight, preferably 20 to 40 % by weight, more preferably 25 to 35 % by weight of the filler, and
10 to 50 % by weight, preferably 20 to 40 % by weight, more preferably 25 to 40 % by weight of the disintegrant.

According to a preferred embodiment the film-coated tablet according to the invention contain on the surface of the tablet core a sub-coating or separating layer. The optional water-soluble separating layer applied on the tablet core may contain film-forming substances generally used for the preparation of film-coated tablets. A preferred water-soluble film-forming substance is hydroxypropyl-methyl cellulose alone or in combination with other water soluble polymers such as polyvinylpyrolidone. Besides the film-forming polymer optionally further auxiliary agents, such as plasticizers, e.g. polyethylene glycol, propylene glycol, titanium dioxide and talc can be used in the coating. The sub-coating is used to separate gastro-resistant coating from direct contact with tablet core.

The sub-coating is preferably applied in an amount of 2 to 20 % by weight, preferably 5 to 15 % by weight (related to the total weight of the composition).

The optional separating layer has preferably a thickness of 20 to 120 µm, more preferably of 50 to 90 µm.

The enteric coating layer preferably comprises film-forming substances such as cellulose acetate phthalate, polyvinylacetate phthalate, hydroxypropyl-methyl cellulose phthalate, hydroxypropyl-methyl cellulose acetate succinate, ethylacrylate methacrylic acid copolymer, methylmethacrylate methacrylic acid copolymer, shellac, more preferably copolymers of methacrylic acid and methacrylic acid esters, such as anionic polymer of methacrylic acid and methacrylates with -COOH groups, e.g. L-form Eudragits. These polymers can be used as organic solutions or preferably as aqueous dispersions, and optionally plasticizers, e.g. triacetin, polyethyleglycole (macrogol) or triethyl citrate, or talc may be added to them. The enteric coating is insoluble in acidic medium. It prevents a disintegration of the tablets in the acidic medium of the stomach which would cause degradation of the pantoprazole and consequently diminishing of therapeutic effect of such the composition.

The enteric coating layer is preferably applied in an amount of 8 to 20 % by weight, more preferably 10 to 15 % by weight (related to the total weight of the composition).

The enteric coating layer has preferably a thickness of 85 to 150 µm or more, more preferably of 90 to 120 µm.

The thickness of the sub-coating and the enteric coating is measured on the scanning electron microscope of the cross-section of coated tablets.

The film-coated tablet according to the invention containing pantoprazole can be prepared as follows. First a tablet core is provided by mixing of the active ingredient, a disintegrant, a filler, an alkalizing agent and any optional components, and compressing the homogenous mixture to tablets without the addition of water. Mixing is preferably performed in a biconical mixer and tablets are preferably formed on a rotary tabletting machine. The thus-obtained tablets are then coated first with a water-soluble coating, then with an enteric coating resistant to gastric acid and finally with a further optional water-soluble coating.

The pantoprazole particles used for preparing the pharmaceutical compositions can be prepared e.g. by crystallization. It is preferred to produce the particles by agglomeration of smaller particles which are commercially available. Agglomeration is performed by wetting pantoprazole with water, passing the wet pantoprazole through a sieve having a mesh size in the range 0.8 to 2 mm, preferably 0.8 to 1.2 mm, more preferably 1.0 mm, and drying the mass is a fluid bed dryer at product temperatures 35 to 50°C.

The water is preferably adjusted to a pH of 10 to 12 by the addition of an alkalizing agent such as sodium hydroxide, sodium carbonate, trisodium phosphate, meglumine or glucosamine. Mixing is preferably performed in a high shear mixer. As the starting material commercially available pantoprazole can be used, preferably pantoprazole having an average particle size of at least 25 µm is used. Drying is preferably performed at a temperature of 35 to 50°C.

According to the process of the present invention granulation of the active ingredient can be achieved without the addition of other ingredients such as sugar alcohols and disintegrants. The granulate of the present invention can be dried in significantly shorter time than granulate prepared according to the prior art e.g. by use of an aqueous solution of mannitol. A granulate of agglomerated pantoprazole sodium sesquihydrate prepared according to the present invention can be dried to a residual moisture content below 1 % by weight in less than 1 hour while the moisture content of a granulate prepared with an aqueous solution of mannitol is just below 3 % by weight after 2 to 3 hours of drying at identical conditions. The residual moisture is determined by a halogen moisture analyzer (Mettler HR 73; 20 min at 85 °C). Thus the present invention provides a granulate containing less moisture than prior art granulates, preferably less than 1 % by weight, and less degradation products since drying of prior art granulates down to a moisture content of less than 1 % by weight unavoidably results in the formation of degradation products. Furthermore, due to the reduced moisture content the granulate shows improved stability during storage.

The agglomerated drug can be used in the preparation of tablet cores either by direct compression or further granulation with excipients such as fillers, stabilizers and disintegrants.

### Examples

### Example 1: Preparation of agglomerated pantoprazole sodium sesquihydrate

6.14 kg of pantoprazole sodium sesquihydrate were transferred into the high sheare mixer (Collet Gral 75) and wetted while mixing with 1.9 kg purified water. The wet mass was transferred to a fluid bed dryer (Glatt WSG 5) and dried with inlet air temperature (60°C) for 25 minutes. After drying the granulate was sieved through a 1.0 mm sieve and dried for an additional 20 minutes. The residual moisture of the dried granulate was 0.6% as determined by a halogen moisture analyzer (Mettler HR 73; 20 min at 85°C).

The average particle size of the product was determined by laser light scattering. 100 to 800 mg of the particles were dispersed in 5 to 8 ml of vegetable oil (sunflower oil). No solubilizers or surfactants were used. Then the obtained suspension was subjected to size determination (Malvern Mastersizer MS 2000). According to the manufacturer's information, the Malvern Mastersizers allows for a measurement of particle size distributions in the range of 20 nm to 2000 µm, with a precision of better than 0.5%. The product had an average particle size of 141 µm. This product was used in the following examples.

### Example 2:

### A) Tablet core

| *Ingredient* | *Amount* |
|---|---|
| Pantoprazole sodium sesquihydrate | 315.7 g |
| (Example 1) | |
| Sodium carbonate | 140.0 g |
| Mannitol (granulated) | 153.3 g |
| Crosspovidone (type A)¹ | 350.0 g |
| Microcrystalline cellulose | 245.0 g |
| (Avicel PH 200) | |
| Magnesium stearate | 21.0 g |

| | |
|---|---|
| ¹ cross-linked polyvinyl pyrrolidone, average particle size < 60 µm | |

All ingredients were mixed in biconical mixer and pressed into tablets on rotary tabletting machine.

### B) Separating layer

| *Ingredient* | *Amount* |
|---|---|
| Hypromellose¹ 3 | 176.0 g |
| cp | |
| Kollidon K25² | 4.4 g |
| Propylenglycole | 44.0 g |
| Water | 1375.0 g |

| | |
|---|---|
| ¹ hydroxypropyl methyl cellulose ² polyvinylpyrrolidone (not cross-linked) | |

Hypromellose and Kollidon were dissolved in water. Propylenglycole was added to the solution. Tablet cores obtained under A were coated by spraying the solution obtained under B onto the tablet cores using a Manesty coating equipment.

### C) Enteric coating

| *Ingredient* | *Amount* |
|---|---|
| Eudragit L-30D¹ | 176.0 g² |
| Triethycitrate | 17.6 g |
| Water | 583.0 g |

| | |
|---|---|
| ¹ anionic polymer of methacrylic acid and methacrylates with a -COOH group ² expressed as dry substance weight | |

Eudragit dispersion was diluted with water and triethylcitrate was dispersed into the diluted Eudragit dispersion. The coating dispersion containing Eudragit L30D, triethylcitrate and water was sprayed onto coated tablets obtained under B using the same equipment as in step B.

### Example 3:

### A) Tablet core

| *Ingredient* | *Amount* |
|---|---|
| Pantoprazole sodium sesquihydrate | 315.7 g |
| (Example 1) | |
| Sodium carbonate | 140.0 g |
| Lactose (spray dryed) | 153.3 g |
| Crosspovidone (Type B) | 350.0 g |
| Microcrystalline cellulose | 245.0 g |
| (Avicel PH 200) | |
| Magnesium stearate | 21.0 g |

All ingredients were mixed in biconical mixer and pressed into tablets on rotary tabletting machine.

### B) Separating layer

| *Ingredient* | *Amount* |
|---|---|
| Hypromellose 3 cp | 176.0 g |
| Kollidon K25 | 4.4 g |
| Propylenglycole | 44.0 g |
| Water | 1375.0 g |

Hypromellose and Kollidon were dissolved in water. Propylenglycole was added to the solution. Tablet cores obtained under A were coated by spraying the solution obtained under B onto the tablet cores in a Manesty coating equipment.

### C) Enteric coating

| *Ingredient* | *Amount* |
|---|---|
| Eudragit L-30D | 211.2 g¹ |
| Macrogole² 6000 | 21.1 g |
| Talc | 79.2 g |
| Water | 700.0 g |

| | |
|---|---|
| ¹ expressed as dry substance weight ² polyethylenglycol | |

Eudragit dispersion was diluted with a part of the water and Macrogole was dissolved in rest of water. Talc was dispersed into the Macrogole solution and the obtained suspension was added to the diluted Eudragit dispersion. The obtained suspension was sprayed onto coated tablets obtained under B using the same equipment as in step B.

### Example 4:

### A) Tablet core

| *Ingredient* | *Amount* |
|---|---|
| Pantoprazole sodium sesquihydrate | 315.7 g |
| (Example 1) | |
| Sodium carbonate anhydrous | 140.0 g |
| Mannitol | 363.3 g |
| Crosspovidone (type A)¹ | 70.0 g |
| Crosspovidone (type B)² | 280.0 g |
| Magnesium stearate | 21.0 g |

| | |
|---|---|
| ¹ cross-linked polyvinyl pyrrolidone, average particle size < 60 µm ² cross-linked polyvinyl pyrrolidone, average particle size > 80 µm | |

All ingredients were mixed in biconical mixer and pressed into tablets on rotary tabletting machine. Loss on drying of the mixture for tabletting: 1,75% (Mettler halogen dryer: 85°C 20 min.)

### B) Separating layer

| *Ingredient* | *Amount* |
|---|---|
| Hypromellose 3 cp | 176.0 g |
| Kollidon K25 | 4.4 g |
| Titanium dioxide | 3.7 g |
| Propylenglycole | 44.0 g |
| Water | 1375.0 g |

Hypromellose and Kollidon were dissolved in water. Propylenglycol was added to the solution. Pigment was homogeneously dispersed into the obtained solution. Tablet cores obtained under A were coated by spraying the suspension obtained under B onto the tablet cores in a Manesty coating equipment.

### C) Enteric coating

| *Ingredient* | *Amount* |
|---|---|
| Eudragit L-30D | 125.4 g¹ |
| Triethycitrate | 12.5 g |
| Macrogole 6000 | 2.6 g |
| Talc | 57.4 g |
| Water | 415.8 g |

| | |
|---|---|
| ¹ expressed as dry substance weight | |

Eudragit dispersion was diluted with a 1^{st} part of water and Macrogole was dissolved in 2^{nd} part of water. Talc was dispersed into the Macrogole solution and the obtained suspension was added to the diluted Eudragit dispersion. Trietylcitrate was dispersed in 3^{rd} part of water and the obtained dispersion was added to the suspension. The obtained coating suspension was sprayed onto coated tablets obtained under B using the same equipment as in step B.

### Example 5:

### A) Tablet core

| *Ingredient* | *Amount* |
|---|---|
| Pantoprazole sodium sesquihydrate | 315.7 g |
| (Example 1) | |
| Sodium carbonate anhydrous | 140.0 g |
| Magnesium carbonate heavy | 14.0 g |
| Sorbitole (granulated) | 349.3 g |
| Crosspovidone (Type B) | 350.0 g |
| Magnesium stearate | 21.0 g |

All ingredients were mixed in biconical mixer and pressed into tablets on rotary tabletting machine.

### B) Separating layer

| *Ingredient* | *Amount* |
|---|---|
| Hypromellose 3 cp | 176.0 g |
| Kollidon K25 | 4.4 g |
| Titanium dioxide | 3.7 g |
| Propylenglycole | 44.0 g |
| Water | 1375.0 g |

Hypromellose and Kollidon were dissolved in water. Propylenglycol ' was added to the solution. Pigment was homogeneously dispersed into the obtained solution. Tablet cores obtained under A were coated by spraying the suspension obtained under B onto the tablet cores in a Manesty coating equipment.

### C) Enteric coating

| *Ingredient* | *Amount* |
|---|---|
| Eudragit L-30D | 146.3 g¹ |
| Triethycitrate | 14.6 g |
| Macrogole 6000 | 3.1 g |
| Talc | 67.0 g |
| Water | 485.0 g |

| | |
|---|---|
| ¹ expressed as dry substance weight | |

Eudragit dispersion was diluted with a 1^{st} part of the water and Macrogole was dissolved in 2^{nd} part of water. Talc was dispersed into the Macrogole solution and the obtained suspension was added to the diluted Eudragit dispersion. Trietylcitrate was dispersed in 3^{rd} part of water and the obtained dispersion was added to the suspension. The obtained coating suspension was sprayed onto coated tablets obtained under B using the same equipment as in step B.

### Example 6:

### A) Tablet core

| *Ingredient* | *Amount* |
|---|---|
| Pantoprazole sodium sesquihydrate | 631,4 g |
| (Example 1) | |
| Sodium carbonate anhydrous | 140,0 g |
| Sorbitole (granulated) | 518,0 g |
| Crospovidone (Type A) | 210,0 g |
| Crospovidone (Type B) | 700,0 g |
| Calcium stearate | 14.0 g |

All ingredients were mixed in biconical mixer and pressed into tablets on rotary tabletting machine.

### B) Separating layer

| *Ingredient* | *Amount* |
|---|---|
| Hypromellose 3 cp | 176.0 g |
| Kollidon K25 | 4.4 g |
| Titanium dioxide | 3.7 g |
| Propylenglycole | 44.0 g |
| Water | 1375.0 g |

Hypromellose and Kollidon were dissolved in water. Propylenglycol was added to the solution. Pigment was homogeneously dispersed into the obtained solution. Tablet cores obtained under A were coated by spraying the suspension obtained under B onto the tablet cores in a Manesty coating equipment.

### C) Enteric coating

| *Ingredient* | *Amount* |
|---|---|
| Eudragit L-30D | 176,0 g¹ |
| Macrogole 6000 | 17,6 g |
| Talc | 66.0 g |
| Water | 583.0 g |

| | |
|---|---|
| ¹ expressed as dry substance weight | |

Eudragit dispersion was diluted with a 1^{st} part of the water and Macrogole was dissolved in 2^{nd} part of water. Talc was dispersed into the Macrogole solution and the obtained suspension was added to the diluted Eudragit dispersion. The obtained coating suspension was sprayed onto coated tablets obtained under B using the same equipment as in step B.

### Example 7:

### A) Tablet core

| *Ingredient* | *Amount* |
|---|---|
| Pantoprazole sodium sesquihydrate¹ | 315.7 g |
| Sodium carbonate anhydrous | 70.0 g |
| Crospovidone (Type A) | 105.0 g |
| Mannitol (kristalline) | 83.3 g |

| | |
|---|---|
| ¹ average particle size 66.0 µm | |

0.3 g sodium carbonate anhydrous was dissolved in 370 g of water to form solution (I). The ingredients listed in the above table were placed into a high shear mixer (Collet grall 10) and solution (I) was slowly added to the mixture while mixing. Kneading was used for 1 min. The wet mass was passed through 18 mesh (1.0 mm) sieve and dried in fluid bed granulator/dryer (Glatt GPCG-3) until the loss on drying was lower than 3%.

To the dry granulate following ingredients were added to obtain a mixture for tabletting:

| *Ingredient* | *Amount* |
|---|---|
| Sorbitole (granulated) | 259,0 g |
| Crosspovidone (Type B) | 350.0 g |
| calcium stearate | 7.0 g |

All ingredients were mixed in biconical mixer and pressed into tablets on rotary tabletting machine.

### B) Separating layer

| *Ingredient* | *Amount* |
|---|---|
| Hypromellose 3 cp | 176.0 g |
| Kollidon K25 | 4.4 g |
| Titanium dioxide | 3.7 g |
| Propylenglycole | 44.0 g |
| Water | 1375.0 g |

Hypromellose and Kollidon were dissolved in water. Propylenglycol was added to the solution. Pigment was homogeneously dispersed into the obtained solution. Tablet cores obtained under A were coated by spraying the suspension obtained under B onto the tablet cores in a Manesty coating equipment.

### C) Enteric coating

| *Ingredient* | *Amount* |
|---|---|
| Eudragit L-30D | 176,0 g¹ |
| Macrogole 6000 | 17,6 g |
| Talc | 66.0 g |
| Water | 583.0 g |

| | |
|---|---|
| ¹ expressed as dry substance weight | |

Eudragit dispersion was diluted with a 1^{st} part of water and Macrogole was dissolved in 2^{nd} part of water. Talc was dispersed into the Macrogole solution and the obtained suspension was added to the diluted Eudragit dispersion. The obtained coating suspension was sprayed onto coated tablets obtained under B using the same equipment as in step B.

### Example 8:

### A) Tablet core

| *Ingredient* | *Amount* |
|---|---|
| Pantoprazole sodium sesquihydrate | 315.7 g |
| (Example 1) | |
| Sodium carbonate anhydrous | 70.0 g |
| Crospovidone (TypeA) | 105.0 g |
| Mannitol (kristalline) | 83.3 g |

0.3 g Anhydrous sodium carbonate was dissolved in 370 g of water to form solution (I). The ingredients listed in the above table were placed into a high shear mixer (Collet grall 10) and solution (I) was slowly added to the mixture while mixing. Kneading was used for 1 min. The wet mass was passed through 18 mesh (1.0 mm) and dried in fluid bed dryer (Glatt GPCG-3) for 3 hours to a residual moisture content of 3% (Halogen moisture analyzer Mettler HR 73; 20 min at 85°C).

To the dry granulate following ingredients were added to obtain a mixture for tabletting:

| *Ingredient* | *Amount* |
|---|---|
| Sorbitole (granulated) | 259,0 g |
| Crosspovidone (Type B) | 350.0 g |
| calcium stearate | 7.0 g |

All ingredients were mixed in biconical mixer and pressed into tablets on rotary tabletting machine.

### B) Separating layer

| *Ingredient* | *Amount* |
|---|---|
| Hypromellose 3 cp | 176.0 g |
| Kollidon K25 | 4.4 g |
| Titanium dioxide | 3.7 g |
| Propylenglycole | 44.0 g |
| Water | 1375.0 g |

Hypromellose and Kollidon were dissolved in water. Propylenglycole was added to the solution. Pigment was homogeneously dispersed into the obtained solution. Tablet cores obtained under A were coated by spraying the suspension obtained under B onto the tablet cores in a Manesty coating equipment.

### C) Enteric coating

| *Ingredient* | *Amount* |
|---|---|
| Eudragit L-30D | 176,0 g¹ |
| Macrogole 6000 | 17,6 g |
| Talc | 66.0 g |
| Water | 583.0 g |

| | |
|---|---|
| ¹ expressed as dry substance weight | |

Eudragit dispersion was diluted with a 1^{st} part of water and Macrogole was dissolved in 2^{nd} part of water. Talc was dispersed into the Macrogole solution and the obtained suspension was added to the diluted Eudragit dispersion. The obtained coating suspension was sprayed onto coated tablets obtained under B using the same equipment as in step B.

## Claims

1. Pharmaceutical composition comprising pantoprazole as the active ingredient, **characterized in that** the pantoprazole is present in the form of particles having an average particle size within the range of 60 µm to 250 µm.

2. The pharmaceutical composition of claim 1, wherein the pantoprazole particles have an average particle size within the range of 100 µm to 250 µm.

3. The composition of claim 1 or 2, wherein the pantoprazole particles have an average particle size within the range of 125 µm to 230 µm.

4. The composition of any one of the preceding claims, wherein the pantoprazole particles have an average particle size within the range of 140 µm to 200 µm.

5. The composition of any one of the preceding claims, wherein the pantoprazole is present in form of the sodium salt.

6. The composition of claim 5, wherein the pantoprazole is present in the form of pantoprazole sodium sesquihydrate.

7. The composition of any one of the preceding claims, wherein the formulation has the form of a tablet comprising
(a) a core comprising the active ingredient, an alkalizing substance, a filler and a disintegrant;
(b) an optional water-soluble separating layer; and
(c) an enteric coating.

8. The composition of claim 7, wherein the alkalizing agent is an alkali or earth alkali hydroxide, an alkali or earth alkali carbonate, an alkali or earth alkali hydrogen carbonate, a phosphate, a glucosamine or a mixture of two or more of these compounds, preferably sodium hydroxide, sodium carbonate, sodium hydrogen carbonate, sodium phosphate or a mixture of two or more of these components, more preferably sodium carbonate.

9. The composition of claim 7 or 8, wherein the filler is lactose or a sugar alcohol, such as mannitol, sorbitol, maltitol, erythrol, or a mixture of two or more of these components.

10. The composition of claim 9, wherein the filler is a mixture of mannitol and sorbitol.

11. The composition of claim 10, wherein the mixture comprises 1 part by weight of mannitol and 1 to 5 parts by weight of sorbitol, preferably 2 to 4 parts by weight of sorbitol and more preferably 2.8 to 3.8 parts by weight of sorbitol

12. The composition of any one of claims 7 to 11, wherein the sugar alcohol has an average particle size within the range of 30 µm to 500 µm.

13. The composition of any one of claims 7 to 12, wherein the disintegrant is crospovidone, sodium starch glycolate, croscarmelose sodium, sodium carboxymethyl starch, sodium carboxymethyl cellulose, potassium carboxymethyl cellulose, or a mixture of two or more of these components.

14. The composition of claim 13, wherein the disintegrant is a mixture of crospovidone having an average particle size within the range of 10 µm to 60 µm and crospovidone having an average particle size within the range of 80 µm to 200 µm.

15. The composition of claim 14, wherein the disintegrant is a mixture of 1 part by weight of crospovidone having an average particle size of 10 to 60 µm and 1 to 5 parts by weight of crospovidone having an average particle size of 80 to 200 µm.

16. The composition of any one of claims 7 to 15, wherein the core additionally comprises a lubricant selected from stearic acid, a hydrogenated vegetable oil, paraffin or an alkali or alkaline earth stearate, aluminum stearate, sodium stearyl fumarate or a mixture of two or more of these compounds, preferably magnesium or calcium stearate, more magnesium potassium stearate.

17. The composition of any one of claims 7 to 16, wherein the core additionally comprises a binder selected from microcrystalline cellulose, hydroxypropyl methyl cellulose, polyvinyl pyrrolidone or a mixtures of two or more of these compounds.

18. The composition of any one of claims 7 to 17, wherein the core comprises
15 to 50 % by weight of the active ingredient,
2 to 25 % by weight of the alkalizing substance,
10 to 50 % by weight of the filler, and
10 to 50 % by weight of the disintegrant.

19. The composition of any one of claims 7 to 18, wherein the optional separating layer comprises a water-soluble film-forming substance, preferably hydroxypropyl methyl cellulose.

20. The composition of any one of claims 7 to 19, wherein the optional separating layer has a thickness of 20 µm to 120 µm.

21. The composition of any one of claims 7 to 20, wherein the enteric coating comprises film-forming substances, preferably cellulose acetate phthalate, polyvinyl- acetate phthalate, hydroxypropyl-methyl cellulose phthalate, hydroxypropyl-methyl cellulose acetate succinate, ethylacrylate methacrylic acid copolymer, methylmethacrylate methacrylic acid copolymer, shellac, preferably copolymers of methacrylic acid and methacrylic acid esters such as anionic polymer of methacrylic acid and methacrylates with -COOH groups.

22. The composition of any one of claims 7 to 21, wherein the enteric coating layer has a thickness of 85 µm to 150 µm.

23. A process for the preparation of pantoprazole having an average particle size within the range of 60 µm to 250 µm comprising the steps of wetting pantoprazole with water in a high shear mixer, passing the wet pantoprazole through a sieve having a mesh size within the range of 0.8 to 2 mm, and drying the mass is a fluid bed dryer.

24. The process of claim 23, wherein drying is performed at a product temperature of 35 to 50 °C.

25. The process of any one of claims 23 and 24, wherein the mass is dried to a residual moisture content of less than 1 % by weight.

26. The process of any one of claims 23 to 25, wherein the water used for wetting has a pH of 10 to 12.

27. The product obtained by the process of any one of claims 23 to 26.
